# EUROPEAN PATENT APPLICATION

(11) **EP 4 556 033 A1**
(43) Date of publication of application: **21.05.2025**
(21) Application number: 23872208.6
(22) Date of filing: 22.09.2023
(51) Int. Cl.: A61M 5/145, A61M 5/168, G06F 3/041

(54) **MEDICAL DEVICE**

(30) Priority: 29.09.2022 JP 2022157045
(71) Applicant: Terumo Kabushiki Kaisha, Tokyo 151-0072 (JP)
(72) Inventor: KOKUBU, Tomotaka, Ashigarakami-gun, Kanagawa 259-0151 (JP); TSUTSUI, Yuichi, Ashigarakami-gun, Kanagawa 259-0151 (JP); TABETA, Keiya, Ashigarakami-gun, Kanagawa 259-0151 (JP)
(74) Representative: KIPA AB
(86) International application number: PCT/JP2023/034592
(87) International publication number: WO 2024/070983

(57) **Abstract**

A medical device according to the present disclosure includes: an operation reception unit that includes an operation reception surface; a position acquisition unit that is capable of acquiring position information based on a position of a detection target on the operation reception surface, the detection target being **in** contact with or **in** proximity to the operation reception surface of the operation reception unit; and a control unit that specifies an operation input by the detection target on a basis of a change **in** the acquired position information. The operation reception surface includes an uneven surface having a sea-island structure **in** which a plurality of protruding surface portions are dispersedly arranged per 1 **mm** square area.

## Description

### Technical Field

The present disclosure relates to a medical device.

### Background Art

There is known a medical device that includes an input device that receives an input operation by an operator, sets a setting value on the basis of the received input operation, and operates on the basis of the setting value. Examples of such a medical device include a syringe pump in which a syringe containing a liquid such as a medicinal solution is placed, and a speed at which a pusher of the placed syringe is pushed out is controlled according to a setting value set by an operator to feed the liquid into a living body such as a patient while controlling a flow rate. A medical device such as a syringe pump includes a touch panel as an input device used for setting a predetermined setting value such as an administration rate, and can change the setting value on the basis of a touch operation performed on the touch panel (see, for example, Patent Literature 1).

### Citation List

### Patent Literature

Patent Literature **1:** JP 6736981 B1

### Summary of Invention

### Technical Problem

At a medical site, an operator may operate a panel surface as an operation reception surface of a touch panel as an operation reception unit of a medical device in a state of wearing a glove instead of a bare hand. Therefore, the operation reception surface of the medical device is preferably configured to be easily operated not only with bare hands but also in a state of wearing a glove.

In addition, a liquid such as a medicinal solution may adhere to the operation reception surface. In a state where the liquid adhering to the operation reception surface is retained, detection accuracy of the operation of the operation reception surface by the operator may be deteriorated by the retained liquid.

An object of the present disclosure is to provide a medical device including an operation reception surface capable of achieving both improvement in operability in a state where a glove is worn and suppression of decrease in detection accuracy of an operation in a state where liquid is attached.

### Solution to Problem

A medical device as a first aspect of the present disclosure includes:
(1) an operation reception unit that includes an operation reception surface;
   a position acquisition unit that is capable of acquiring position information based on a position of a detection target on the operation reception surface, the detection target being in contact with or in proximity to the operation reception surface of the operation reception unit; and
   a control unit that specifies an operation input by the detection target on a basis of a change in the acquired position information, in which
   the operation reception surface includes an uneven surface having a sea-island structure in which a plurality of protruding surface portions are dispersedly arranged per 1 mm square area.
   In the medical device according to (1) as one embodiment of the present disclosure,
(2) the position acquisition unit is a capacitive position detection sensor capable of acquiring the position information of the detection target by detecting a change in capacitance caused by contact or proximity of the detection target to the operation reception surface.
   In the medical device according to (1) or (2) as one embodiment of the present disclosure,
(3) an area ratio of the protruding surface portion at a cross-sectional position of a height of 50% or more of the uneven surface per area of a 300 µm square of the uneven surface is more than 0% and 60% or less, and
   the height of 50% or more means a position of 50% or more in a height direction in a case where a lowest position is 0% and a highest position is 100% in the height direction orthogonal to the uneven surface.
   In the medical device according to any one of (1) to (3) as one embodiment of the present disclosure,
(4) an area ratio of the protruding surface portion at a cross-sectional position of a height of 70% or more of the uneven surface per area of a 300 µm square of the uneven surface is more than 0% and 20% or less, and
   the height of 70% or more means a position of 70% or more in a height direction in a case where a lowest position is 0% and a highest position is 100% in the height direction orthogonal to the uneven surface.
   In the medical device according to any one of (1) to (4) as one embodiment of the present disclosure,
(5) an arithmetic average height of the uneven surface is 0.82 to 1.44 µm.
   The medical device according to any one of (1) to (5) as one embodiment of the present disclosure includes:
(6) a housing that accommodates the position acquisition unit and the control unit, in which
   an outer surface of the housing includes an inclined surface portion extending obliquely with respect to a horizontal direction so as to face an upper side in a vertical direction in a use state, and
   the operation reception surface of the operation reception unit is exposed from the inclined surface portion of the housing and extends along the inclined surface portion.
   In the medical device according to any one of (1) to (6) as one embodiment of the present disclosure,
(7) the operation reception surface includes an annular groove having an arcuate cross-sectional shape orthogonal to an extending direction, and
   the uneven surface is formed on an inner surface of the annular groove.

### Advantageous Effects of Invention

According to the present disclosure, it is possible to provide a medical device including an operation reception surface capable of achieving both improvement in operability in a state where a glove is worn and suppression of decrease in detection accuracy of an operation in a state where liquid is attached.

### Brief Description of Drawings

Fig. 1 is a perspective view of a syringe pump as an embodiment of a medical device according to the present disclosure.
Fig. 2 is a block diagram illustrating a configuration of a syringe pump illustrated in Fig. 1.
Fig. 3 is a diagram illustrating an example of arrangement of detection regions on an operation reception surface of the syringe pump illustrated in Fig. 1.
Fig. 4 is a flowchart illustrating setting value increase/decrease processing executed by the syringe pump illustrated in Fig. 1.
Fig. 5 is an enlarged cross-sectional view illustrating the vicinity of an operation reception unit in a cross-sectional view at a position of the operation reception unit in the syringe pump illustrated in Fig. 1 in an enlarged manner.
Fig. 6 is an enlarged view illustrating a part of the operation reception surface of the operation reception unit.
Fig. 7 is a diagram illustrating a cross-sectional shape of the operation reception surface of the operation reception unit.
Fig. 8 illustrates an experimental result of an experiment for evaluating the influence of the glove operability and the liquid discreteness depending on the arithmetic average height of the uneven surface of the operation reception surface.

### Description of Embodiments

Hereinafter, embodiments of a medical device according to the present disclosure will be described by way of example with reference to the drawings. In the drawings, the same components are denoted by the same reference numerals.

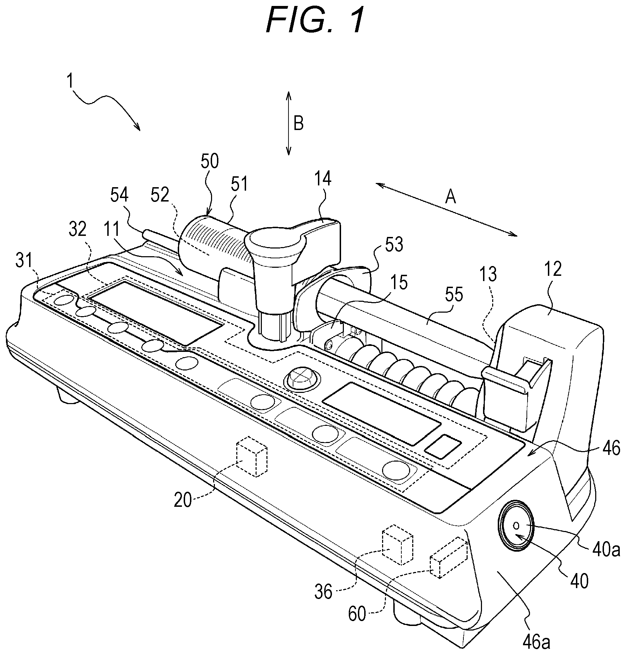

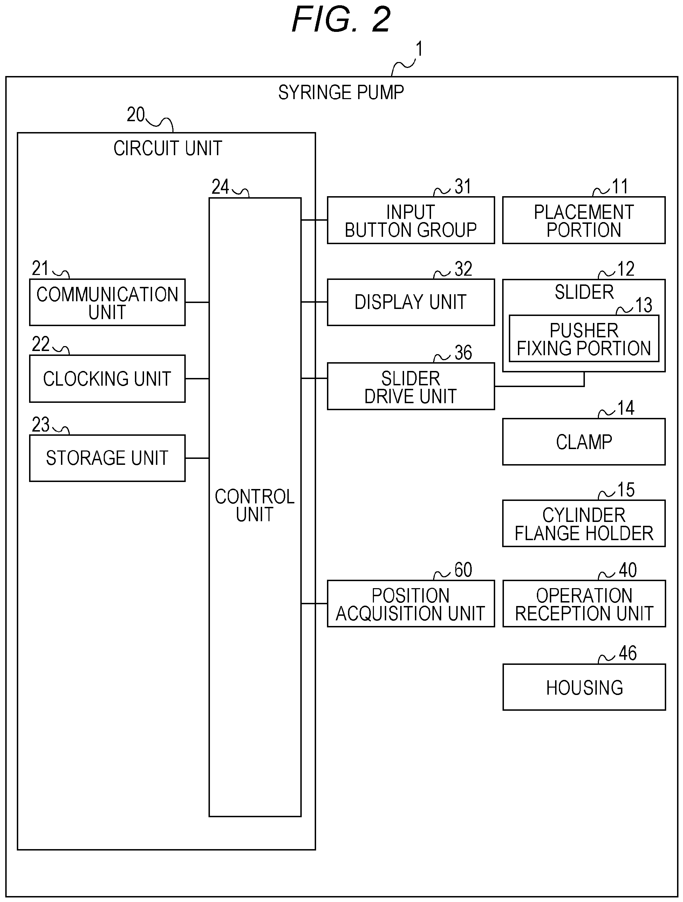

Fig. 1 is a perspective view of a syringe pump 1 as an embodiment of a medical device according to the present disclosure. Fig. 1 illustrates the syringe pump 1 with a syringe 50 placed thereon. As illustrated in Fig. 1, the syringe pump 1 is configured as a pump for feeding a liquid stored in a hollow portion 52 of the syringe 50. Fig. 2 is a block diagram illustrating a configuration of the syringe pump 1. In the present embodiment, the syringe pump 1 as a medical device will be described as an example, but the medical device according to the present disclosure is not limited to the syringe pump.

As illustrated in Figs. 1 and 2, the syringe pump 1 includes a placement portion 11, a slider 12, a clamp 14, a cylinder flange holder 15, a circuit unit 20, an input button group 31, a display unit 32, a slider drive unit 36, an operation reception unit 40, a housing 46, and a position acquisition unit 60.

As illustrated in Fig. 1, the syringe 50 can be placed on the placement portion 11. The syringe 50 placed on the placement portion 11 includes a cylindrical cylinder 51 that defines the hollow portion 52 therein, and a pusher 55 that is inserted into the hollow portion 52 from the proximal end side of the cylinder 51 and is movable in the hollow portion 52 along an extending direction A of the cylinder 51 (hereinafter, it is simply referred to as an "extending direction A") while being in close contact with the inner peripheral surface of the cylinder 51 in the circumferential direction without any gap. The cylinder 51 has a cylinder flange 53 at a proximal end, and defines an outlet hole 54 that communicates the hollow portion 52 with the outside at a distal end. A flexible tube can be connected to the distal end of the cylinder 51. When the tube is connected to the distal end of the cylinder 51, the outlet hole 54 communicates with the flow path in the tube. The hollow portion 52 of the syringe 50 contains a liquid such as a medicinal solution. Hereinafter, in the extending direction A, a direction from the proximal end side to the distal end side of the cylinder 51 may be referred to as "distal end side in the extending direction A", and a direction from the distal end side to the proximal end side of the cylinder 51 may be referred to as "proximal end side in the extending direction A".

As illustrated in Fig. 1, the cylinder 51 of the syringe 50 can be placed on the placement portion 11. As illustrated in Fig. 1, the cylinder flange holder 15 houses a part of the cylinder flange 53 when the cylinder 51 of the syringe 50 is placed on the placement portion 11. As a result, the position of the cylinder 51 with respect to the syringe pump 1 is fixed.

As illustrated in Fig. 1, the slider 12 includes a pusher fixing portion 13. The slider 12 is movable to engage with the pusher 55 of the syringe 50 placed on the placement portion 11. Specifically, the slider 12 is movable along the extending direction A at a position on the proximal end side in the extending direction A with respect to the pusher 55 of the syringe 50 placed on the placement portion 11. The slider 12 fixes the pusher 55 of the syringe 50 placed on the placement portion 11 by the pusher fixing portion 13. In a state where the pusher 55 is fixed to the slider 12 by the pusher fixing portion 13, the pusher 55 moves integrally with the slider 12 along with the movement of the slider 12 in the extending direction A. At this time, the cylinder 51 placed on the placement portion 11 is fixed in the extending direction A with respect to the syringe pump 1 by the cylinder flange holder 15. Therefore, when the slider 12 moves to the distal end side of the syringe 50, the pusher 55 moves to the distal end side with respect to the cylinder 51, and the liquid stored in the hollow portion 52 is discharged from the outlet hole 54. Therefore, the liquid stored in the hollow portion 52 can be fed into the living body through a flow path defined by a tube connectable to the distal end of the cylinder 51.

As illustrated in Fig. 1, the clamp 14 is movable along a direction B orthogonal to the extending direction A, and can fix the cylinder 51 of the placed syringe 50 so as to be sandwiched between the placement portion 11 and the clamp. By fixing the cylinder 51 with the clamp 14, a part of the cylinder flange 53 is hardly detached from the cylinder flange holder 15, so that the cylinder 51 is firmly fixed to the syringe pump 1.

As illustrated in Fig. 2, the circuit unit 20 includes a communication unit 21, a clocking unit 22, a storage unit 23, and a control unit 24.

The communication unit 21 includes a communication interface that transmits and receives information to and from an information processing device such as an external computer by wireless communication or wired communication. The communication interface is, for example, an interface compatible with a mobile communication standard such as **LTE,** 4G standard, or 5G standard, an interface compatible with short-range wireless communication such as Bluetooth (registered trademark), an interface compatible with a wireless LAN communication standard such as IEEE 802.11, or an interface compatible with a wired LAN communication standard such as Ethernet (registered trademark). The "LTE" is an abbreviation for long term evolution. The "4G" is an abbreviation for 4th generation. The "5G" is an abbreviation for 5th generation. "IEEE" is an abbreviation for Institute of Electrical and Electronics Engineers.

The clocking unit 22 measures time and clocks time. The clocking unit 22 may be realized by, for example, a real time clock (RTC). The clocking unit 22 may be realized as one function of the control unit 24.

The storage unit 23 includes, for example, a storage device, and stores various types of information and programs. Specifically, the storage unit 23 stores a program for executing setting value increase/decrease processing executed by the control unit 24, various types of input support processing, and the like. The storage unit 23 stores information of a predetermined setting value (hereinafter, it is simply referred to as a "setting value".) such as the flow rate and the dose of the liquid fed by the syringe pump 1, a control program for driving the slider drive unit 36 based on the setting value and feeding the liquid, and the like. The storage unit 23 stores one of a first circumferential direction and a second circumferential direction as a temporary storage direction. Details of the temporary storage direction stored in the storage unit 23 will be described later. The storage unit 23 includes at least one semiconductor memory, at least one magnetic memory, at least one optical memory, or any combination thereof. The semiconductor memory is, for example, a RAM, a ROM, or a flash memory. The "RAM" is an abbreviation for a random access memory. "ROM" is an abbreviation for read only memory. The RAM is, for example, an SRAM or a DRAM. "SRAM" is an abbreviation for static random access memory. "DRAM" is an abbreviation for dynamic random access memory. The ROM is, for example, an EEPROM. The "EEPROM" is an abbreviation for an electrically erasable programmable read only memory. The flash memory is, for example, an SSD. The "SSD" is an abbreviation for a solid-state drive. The magnetic memory is, for example, an HDD. The "HDD" is an abbreviation for a hard disk drive.

The control unit 24 includes, for example, at least one processor, at least one programmable circuit, at least one dedicated circuit, or an arbitrary combination thereof that implements a predetermined function by reading predetermined information and a program among various information and programs stored in the storage unit 23. Then, the control unit 24 controls the operation of the entire syringe pump 1. The processor is a general-purpose processor such as a CPU or GPU, or a dedicated processor specialized for specific processing. "CPU" is an abbreviation for central processing unit. "GPU" is an abbreviation for graphics processing unit. The programmable circuit is, for example, an FPGA. "FPGA" is an abbreviation for field-programmable gate array. The dedicated circuit is, for example, an ASIC. The "ASIC" is an abbreviation for an application specific integrated circuit. As will be described later, the control unit 24 specifies an operation input from an operation reception surface 40a of the operation reception unit 40 by a detection target such as the operator's fingertip. Specifically, the control unit 24 reads predetermined information and a program stored in the storage unit 23, and executes processing such as setting value increase/decrease processing and various types of input support processing. The control unit 24 transmits and receives information to and from an external information processing device via the communication unit 21. The control unit 24 executes various types of processing on the basis of the information input from the input button group 31 and the position acquisition unit 60, and outputs information accompanying the execution of the various types of processing from the display unit 32.

As illustrated in Fig. 1, the input button group 31 includes various operation buttons disposed on the surface of the housing 46 and capable of receiving an input operation by an operator. The input button group 31 includes, for example, a power button for switching on/off of the operation power of the syringe pump 1, a start button for starting liquid feeding, and a stop button for stopping liquid feeding. The input button group 31 outputs the input information to the control unit 24.

As illustrated in Fig. 1, the display unit 32 includes, for example, a display device such as a liquid crystal display or an organic EL display. The display unit 32 displays a setting value and an actual measurement value of the flow rate of the liquid to be fed, a setting value and an actual measurement value of the dose of the liquid to be fed, various alarm information, and the like on the basis of a signal from the control unit 24.

The slider drive unit 36 includes, for example, a motor, and moves the slider 12 along the extending direction A (see Fig. 1) based on a signal from the control unit 24.

As illustrated in Fig. 1, the operation reception unit 40 is disposed so as to be at least partially exposed to the outside of the syringe pump **1.** More specifically, the operation reception unit 40 includes the operation reception surface 40a exposed from the housing 46. Details of the operation reception unit 40 will be described later.

The position acquisition unit 60 can acquire position information based on the position on the operation reception surface 40a of the detection target such as the fingertip of the operator in contact with or in proximity to the operation reception surface 40a of the operation reception unit 40. The position acquisition unit 60 acquires position information over time. Specifically, the position acquisition unit 60 acquires the position information at a predetermined acquisition timing. The predetermined acquisition timing occurs, for example, at a predetermined cycle. The predetermined acquisition timing may occur, for example, every 10 milliseconds. In this case, the position acquisition unit 60 acquires the position information every 10 milliseconds. However, the predetermined cycle is not limited to 10 milliseconds. The predetermined cycle may be appropriately determined according to the use, specification, or the like of the operation reception unit 40. However, the position acquisition unit 60 preferably acquires the position information every 5 milliseconds to 100 milliseconds, and more preferably acquires the position information every 5 milliseconds to 50 milliseconds. By setting the time interval at which the position acquisition unit 60 acquires the position information to be equal to or less than the upper limit of the above range, for example, in a case where the position of the detection target is quickly changed, it is possible to reduce the possibility that the control unit 24 erroneously recognizes that the direction in which the detection target has actually rotated has rotated in the opposite direction. The position acquisition unit 60 outputs the acquired position information to the control unit 24.

The position acquisition unit 60 of the present embodiment is a capacitive position detection sensor capable of acquiring position information of the detection target by detecting a change in capacitance caused by contact or proximity of the detection target to the operation reception surface 40a. For example, the position acquisition unit 60 may include a plurality of electrodes corresponding to a plurality of detection regions on the operation reception surface 40a, and may acquire position information of the detection target on the operation reception surface 40a based on a change in capacitance between the detection target and each electrode in a case where the detection target of the operator comes into contact with the operation reception surface 40a.

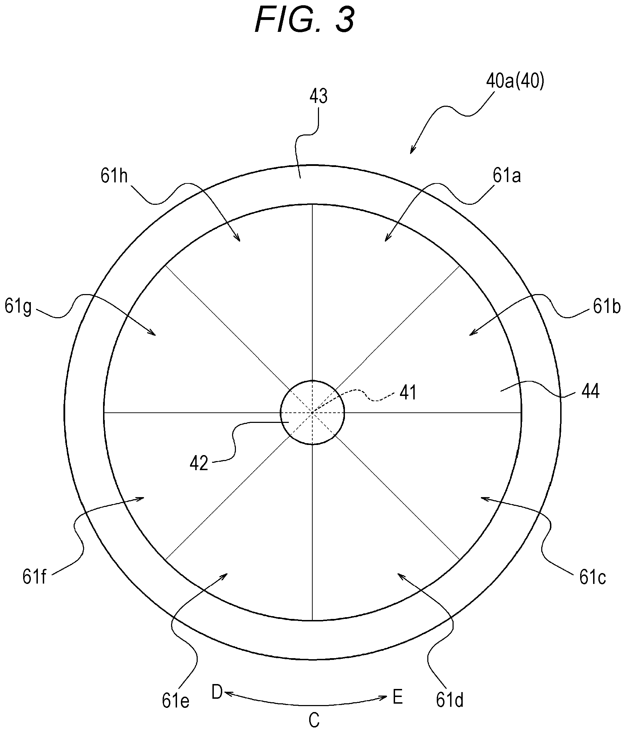

Fig. 3 is a diagram illustrating an example of the arrangement of the detection regions on the operation reception surface 40a. As illustrated in Fig. 3, the position acquisition unit 60 of the present embodiment can detect a plurality of detection regions, for example, eight fan-shaped detection regions 61a to 61h, arranged without a gap along a circumferential direction C around a central portion 41 on the operation reception surface 40a.

The position acquisition unit 60 acquires, as the position information of the detection target, the detection region that the detection target touches or comes closest to among the plurality of detection regions 61a to 61h. Specifically, for example, the position acquisition unit 60 may acquire information indicating the position of the detection target in the three-dimensional space, and acquire a detection region where the position is closest as the position information of the detection target. In addition, the position acquisition unit 60 may be configured with, for example, a detection unit such as a touch pad capable of detecting contact of the detection target in each of a plurality of detection regions, and acquire the detection region in which the contact has been detected as the position information of the detection target. The position acquisition unit 60 outputs information of the detection region as the acquired position information to the control unit 24. Hereinafter, the position information of the detection target acquired by the position acquisition unit 60 will be referred to as "acquired position information", and the detection region acquired as the position information of the detection target by the position acquisition unit 60 will be referred to as "acquired detection region".

As described above, the syringe pump 1 as the medical device of the present embodiment includes the operation reception unit 40 including the operation reception surface 40a, the position acquisition unit 60 capable of acquiring the position information based on the position on the operation reception surface 40a of the detection target in contact with or in proximity to the operation reception unit 40, and the control unit 24 that specifies the operation input by the detection target on the basis of the change in the position information acquired by the position acquisition unit 60. As a result, since the syringe pump 1 does not need to be provided with a rotating member that physically rotates as an input device, it is possible to suppress deterioration of operability even if foreign matter such as liquid adheres. Furthermore, since it is not necessary to provide a rotating member, the syringe pump 1 can have a configuration with a small gap. Therefore, cleaning performance can be improved.

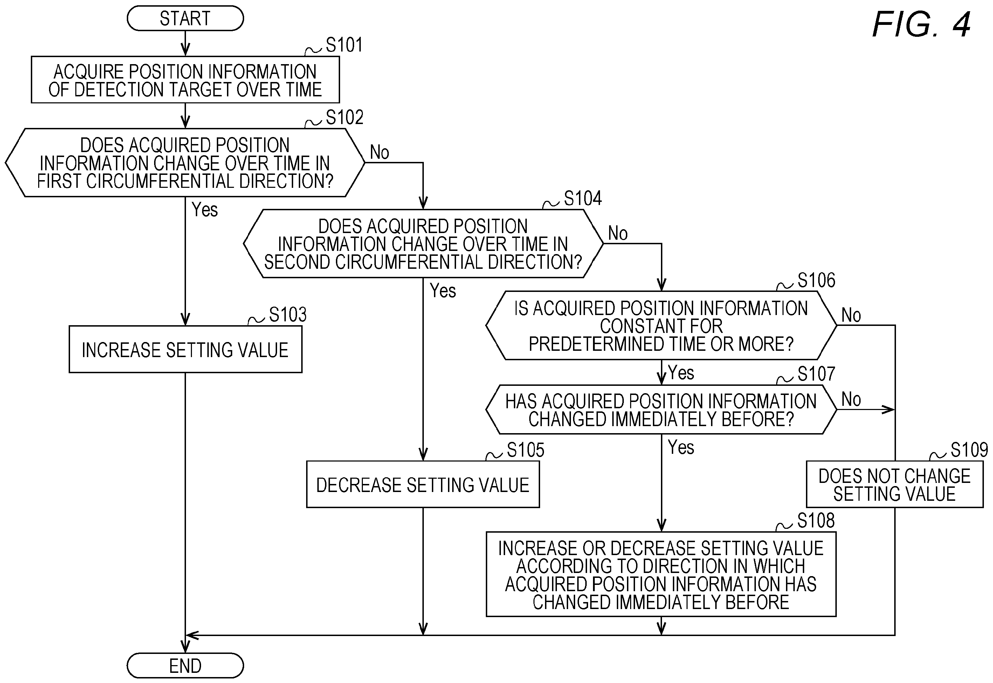

Next, an example of processing of the syringe pump 1 will be described with reference to Fig. 4. Fig. 4 is a flowchart illustrating the setting value increase/decrease processing executed by the syringe pump **1.** As illustrated in Fig. 4, first, the syringe pump 1 acquires position information of the detection target over time using the position acquisition unit 60 (step S101). Specifically, the position acquisition unit 60 acquires the position information of the detection target every predetermined unit time, for example, every 50 milliseconds, and outputs the acquired position information to the control unit 24.

Thereafter, the control unit 24 specifies the operation input by the detection target on the basis of the temporal change in the acquired position information. The control unit 24 increases or decreases the setting value on the basis of, for example, a time change in the circumferential direction C (see Fig. 3) of the acquired detection region. Specifically, the control unit 24 executes processing of the following steps S102 to S107.

The control unit 24 determines whether the acquired position information has a temporal change in a first circumferential direction D (see Fig. 3), which is one direction of the circumferential direction C (step S102). Specifically, the control unit 24 determines whether the acquired detection region changes with time in the first circumferential direction D between two detection regions adjacent to each other, that is, for example, whether the acquired detection region changes with time from the detection region 61a to the detection region 61b.

In a case where it is determined that the acquired position information has a temporal change in the first circumferential direction D (Yes in step S102), the control unit 24 increases the setting value (step S103). On the other hand, in a case where it is determined that the acquired position information does not change with time in the first circumferential direction D (No in step S102), the control unit 24 proceeds to the process of step S104.

The control unit 24 determines whether the acquired position information has a temporal change in a second circumferential direction E (see Fig. 3) that is the other side in the circumferential direction C, that is, the direction opposite to the first circumferential direction D (step S104). Specifically, the control unit 24 determines whether the acquired detection region changes with time in the second circumferential direction E between two detection regions adjacent to each other, that is, for example, whether the acquired detection region changes with time from the detection region 61a to the detection region 61h.

In a case where it is determined that the acquired position information has a temporal change in the second circumferential direction E (Yes in step S104), the control unit 24 decreases the setting value (step S105). On the other hand, in a case where it is determined that the acquired position information does not change with time in the second circumferential direction E (No in step S104), the control unit 24 proceeds to the process of step S106.

The control unit 24 determines whether the acquired position information is constant for a predetermined time or more (step S106). The predetermined time used in the process of step S106 is stored in advance in the storage unit 23, and is, for example, 1 second.

In a case where it is determined that the acquired position information is constant for the predetermined time or more (Yes in step S106), the control unit 24 determines whether the acquired position information has changed immediately before (step S107). In other words, in the process of step S107, the control unit 24 determines whether the acquired position information up to immediately before the first acquired position information used as the basis of the determination in the process of step S106 has changed. At this time, for example, in a case where there is a period in which the position acquisition unit 60 cannot acquire the position information of the detection target because the detection target is once separated from the operation reception unit 40, the previously acquired position information is reset by second input support processing to be described later. Therefore, in this case, only the position information of the detection target continuously acquired again by the position acquisition unit 60 is the determination target in the process of step S107.

In a case where it is determined that the acquired position information has changed immediately before (Yes in step S107), the control unit 24 increases or decreases the setting value according to the direction in which the acquired position information has changed with time immediately before among the first circumferential direction D and the second circumferential direction E (step S108). Specifically, in a case where the direction in which the acquired position information has changed with time immediately before is the first circumferential direction D, the control unit 24 increases the setting value. On the other hand, in a case where the direction in which the acquired position information has changed with time immediately before is the second circumferential direction E, the control unit 24 decreases the setting value. Thereafter, the control unit 24 continues to increase or decrease the setting value during a period in which the acquired position information is constant.

In a case where it is determined that the acquired position information is not constant for the predetermined time or more (No in step S106), or in a case where it is determined that the acquired position information has not changed immediately before (No in step S107), the control unit 24 does not change the setting value (step S109).

As described above, in a case where the acquired position information is constant for a predetermined time or more and the acquired position information has changed immediately before, the syringe pump 1 increases or decreases the setting value according to the direction in which the acquired position information has changed with time immediately before. Therefore, the operator can continue to increase or decrease the setting value according to the direction in which the detection target has been moved immediately before, without continuing to move the detection target.

In the process of step S108, the control unit 24 may increase or decrease the setting value by a constant change amount per unit time. As a result, the operator can estimate the remaining time for the setting value to reach the target value by checking the setting value that changes at a constant rate on the display unit 32, so that the setting value can be easily set to the target value.

In the process of step S108, the control unit 24 may increase or decrease the setting value by the change amount per unit time according to the change amount per unit time of the position information that has changed immediately before. In other words, the control unit 24 may determine the change amount per unit time of the setting value according to the change amount per unit time of the position information that has changed immediately before. Specifically, the control unit 24 may increase the change amount per unit time of the setting value as the change amount per unit time of the position information that has changed immediately before is larger. As described above, since the syringe pump 1 determines the change rate of the setting value according to the change speed of the position information that has changed immediately before, the syringe pump 1 can change the setting value at a rate corresponding to the operation immediately before the operator.

Although Fig. 4 illustrates an example of the processing of the syringe pump 1 based on the operation performed by the operator using the operation reception unit 40, the processing of the syringe pump 1 based on the operation performed by the operator using the operation reception unit 40 is not limited to the above-described processing.

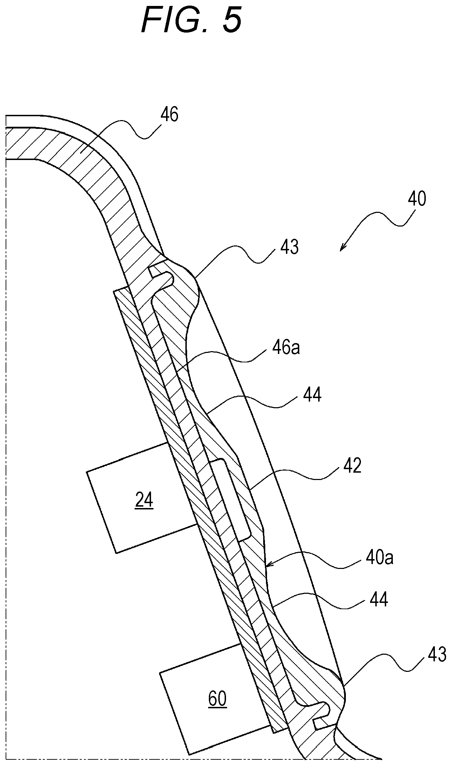

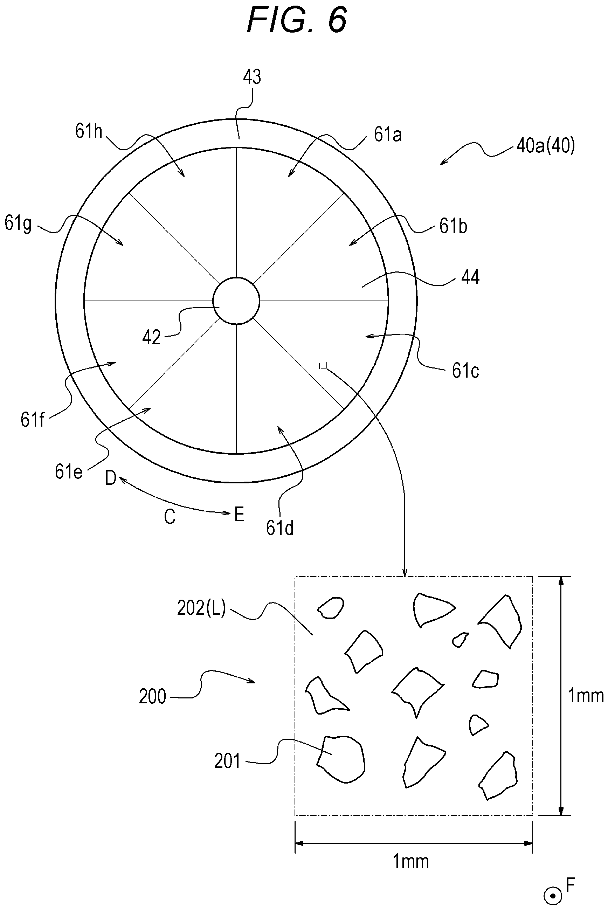

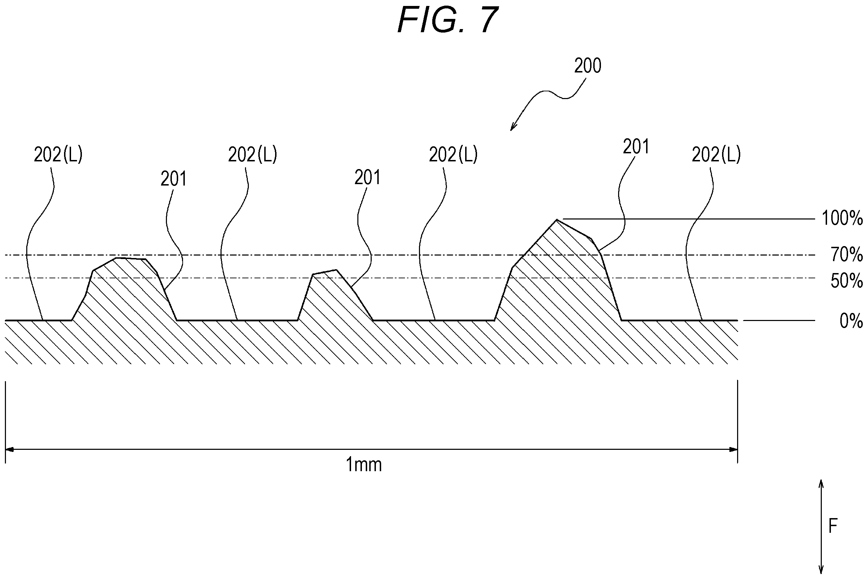

Next, the operation reception surface 40a of the operation reception unit 40 will be described in detail with reference to Figs. 1, 3, and 5 to 7. Fig. 5 is an enlarged cross-sectional view illustrating the vicinity of the operation reception unit 40 in the cross-sectional view at the position of the operation reception unit 40 in the syringe pump 1 in an enlarged manner. Fig. 6 is an enlarged view illustrating a part of the operation reception surface 40a of the operation reception unit 40. Fig. 7 is a diagram illustrating a cross-sectional shape of the operation reception surface 40a of the operation reception unit 40.

As illustrated in Figs. 1, 3, and 5, the operation reception surface 40a of the operation reception unit 40 of the present embodiment includes an annular groove 44. The annular groove 44 is an endless groove extending in the circumferential direction C. In addition, the cross-sectional shape of the annular groove 44 of the present embodiment orthogonal to the circumferential direction C which is the extending direction is an arc shape. Here, the "arc shape" is not limited to a curve connecting two points on the same circumference, but means a concave curve. The operator can perform an operation such as setting value increase/decrease processing (see Fig. 4) by moving the fingertip as the detection target along the circumferential direction C on the inner surface of the annular groove 44. Since the operation reception surface 40a includes the above-described annular groove 44, the fingertip of the operator as the detection target can be arranged so as to be fitted in the annular groove 44, and the fingertip can be easily moved along the annular groove 44. In addition, by forming the cross-sectional shape of the annular groove 44 into the arc shape, the inner surface of the annular groove 44 easily comes into contact with the ball of the finger in a wide range along the ball of the finger of the operator as compared with the case where the cross-sectional shape is a rectangular shape. That is, the fingertip is easily positioned in the annular groove 44. In this manner, since the operation reception surface 40a includes the above-described annular groove 44, operability of the operation reception surface 40a can be enhanced. Although details will be described later, an uneven surface 200 (see Figs. 6 and 7) of the operation reception surface 40a is formed on at least a part of the inner surface of the annular groove 44.

More specifically, the operation reception surface 40a of the present embodiment includes a central protruding portion 42, a peripheral wall 43, and an annular groove 44. The central protruding portion 42 protrudes at a position corresponding to the central portion 41. The peripheral wall 43 is separated radially outward from the central protruding portion 42 and protrudes so as to surround the central protruding portion 42. The peripheral wall 43 extends along the circumferential direction C around the central protruding portion 42. The annular groove 44 is located between the central protruding portion 42 and the peripheral wall 43. In the present embodiment, the edge of the peripheral wall 43 protrudes outward from the outer surface of the housing 46, but the present invention is not limited to this configuration. The edge of the peripheral wall 43 may be flush with the outer surface of the housing 46. The operation reception unit 40 is preferably an integrally molded product with the housing 46 from the viewpoint of suppressing deterioration in operability and improving cleaning performance.

The position acquisition unit 60 does not detect the position information of the detection target in the vicinity of the central portion 41 by the central protruding portion 42. Therefore, the position acquisition unit 60 can suppress simultaneous acquisition of a plurality of detection regions not adjacent in the circumferential direction C as acquired detection regions.

As illustrated in Fig. 1, the housing 46 is an exterior member of the syringe pump 1 that accommodates the position acquisition unit 60, the control unit 24, and the like. As illustrated in Fig. 1, the outer surface of the housing 46 of the present embodiment includes an inclined surface portion 46a. The inclined surface portion 46a is a surface extending so as to be inclined with respect to the horizontal direction so as to face upward in the vertical direction in the use state of the syringe pump 1. The use state of the syringe pump 1 means, for example, a state in which the syringe pump 1 is in a usable posture, such as a state in which the syringe pump 1 is fixed to a stand. In Fig. 1, the posture of the syringe pump 1 in which the upper side in the direction B orthogonal to the extending direction A is the upper side in the vertical direction is the use state of the syringe pump 1. As illustrated in Figs. 1 and 5, the operation reception surface 40a of the operation reception unit 40 is exposed from the inclined surface portion 46a of the housing 46 and extends along the inclined surface portion 46a. In this way, the operator can easily operate the operation reception surface 40a from the upper side in the vertical direction, and the operability of the operation reception surface 40a by the operator can be improved.

Next, details of the operation reception surface 40a will be described with reference to Figs. 6 and 7. As illustrated in Figs. 6 and 7, the operation reception surface 40a includes an uneven surface 200 having a sea-island structure in which a plurality of protruding surface portions 201 are dispersedly arranged per 1 mm square area. The phrase "the plurality of protruding surface portions 201 are dispersedly arranged per 1 mm square area" as used herein means that at least a part of each of at least two or more protruding surface portions 201 is dispersedly arranged per 1 mm square area. That is, at least two or more protruding surface portions 201 may not be entirely included per 1 mm square area. The uneven surface 200 of the present embodiment is formed on the inner surface of the annular groove 44. More specifically, the uneven surface 200 of the present embodiment is formed over the entire recessed surface integrally forming the groove wall surface and the bottom surface of the annular groove 44. As illustrated in Figs. 6 and 7, the uneven surface 200 includes a plurality of protruding surface portions 201 formed in independent protrusions, and a recessed surface portion 202 continuous around the plurality of protruding surface portions 201. In other words, the recessed surface portion 202 forms a continuous reference surface **L.** Here, an uneven surface in which a plurality of hole-shaped independent recessed surface portions are formed with respect to a protruding surface portion as a continuous reference surface may be referred to as a "satin-like surface". On the other hand, the above-described concavo-convex relationship of the uneven surface 200 is opposite to the concavo-convex relationship of the satin-like surface. In other words, the uneven surface 200 is a surface similar to a surface formed by transferring the satin-like surface. Therefore, hereinafter, for convenience of description, the uneven surface 200 of the present embodiment may be referred to as a "transferred satin-like surface", but the method for forming the uneven surface 200 is not limited to transfer of the satin-like surface.

As described above, since the operation reception surface 40a includes the uneven surface 200 having the sea-island structure described above, it is possible to achieve both improvement in operability in a state where a glove is worn and suppression of decrease in detection accuracy of an operation in a state where liquid is attached.

Specifically, examples of the material of the glove used in the medical field include polyvinyl chloride, polyethylene, nitrile, and natural rubber latex. Since the operation reception surface 40a includes the uneven surface 200 having the sea-island structure described above, the contact area with the fingertip of the operator as the detection target can be reduced as compared with a configuration in which the operation reception surface 40a includes only a smooth surface without including the uneven surface 200. Therefore, even when the operator wears the above-described resin or rubber glove, the contact area with the glove can be reduced, and the sliding resistance between the operation reception surface 40a and the glove can be reduced. This improves operability in a state where the glove is worn.

Further, as described above, the uneven surface 200 is a transfer satin-like surface, and has a sea-island structure in which a plurality of protruding surface portions 201 are dispersedly arranged per 1 mm square area. Therefore, the droplets of the liquid such as the medicinal solution adhering to the top of the protruding surface portion 201 are discretely dispersed on the uneven surface 200. Thus, the droplets can be prevented from coalescing and spreading on the uneven surface 200. As a result, the liquid adhering to the uneven surface 200 becomes an unintended conductive path, and it is possible to prevent the position acquisition unit 60 from erroneously detecting the position information of the detection target. That is, even in a state where liquid adheres to the operation reception surface 40a, it is possible to suppress a decrease in detection accuracy of the operation of the operation reception surface 40a by the operator. The shortest distance between the apexes of the adjacent protruding surface portions 201 on the uneven surface 200 may be, for example, 50 µm to 500 µm. Examples of the material for forming the uneven surface 200 include polycarbonate, PC-ABS, ABS resin, and ASA resin.

In particular, as illustrated in Fig. 7, the area ratio of the protruding surface portion 201 at a cross-sectional position of a height of 50% or more of the uneven surface 200 per 300 µm square area of the uneven surface 200 (hereinafter, for convenience of description, simply referred to as "an area ratio of 50% or more of the protruding surface portion 201") is preferably more than 0% and 60% or less. Here, "height of 50%" means a position of 50% in a height direction F when the lowest position in the height direction F orthogonal to the uneven surface 200 is 0% and the highest position is 100%. Therefore, the "height of 50% or more" means any position from the 50% position to the 100% position described above. In a case where the uneven surface 200 is formed as a curved surface, the above-described height direction F means a normal direction at a center point in a 300 µm square region of the uneven surface 200. The "cross-sectional position at a height of 50% or more of the uneven surface 200" means a cross-sectional position orthogonal to the height direction F at an arbitrary position at a height of 50% or more of the uneven surface 200.

By setting the area ratio to 50% or more of the protruding surface portion 201 as described above, the protruding surface portion 201 at the position of 50% to 100% of the height of the uneven surface 200 can be appropriately dispersed to such an extent that the droplets adhering to the top of the protruding surface portion 201 do not coalesce. As a result, even in a state where liquid adheres to the operation reception surface 40a, it is possible to further suppress a decrease in detection accuracy of the operation of the operation reception surface 40a by the operator.

From the viewpoint of improving the discreteness of the liquid adhering to the uneven surface 200, the area ratio of 50% or more of the protruding surface portion 201 is more than 0%, more preferably 40% or less, and still more preferably 20% or less.

Further, as illustrated in Fig. 7, the area ratio of the protruding surface portion 201 at a cross-sectional position of a height of 70% or more of the uneven surface 200 per 300 µm square area of the uneven surface 200 (hereinafter, for convenience of description, simply referred to as "an area ratio of 70% or more of the protruding surface portion 201") is preferably more than 0% and 20% or less. In this way, even in a state where liquid adheres to the operation reception surface 40a, it is possible to further suppress a decrease in detection accuracy of the operation of the operation reception surface 40a by the operator.

From the viewpoint of improving the discreteness of the liquid adhering to the uneven surface 200, the area ratio of 70% or more of the protruding surface portion 201 is more than 0%, more preferably 15% or less, and still more preferably 5% or less.

In particular, as described above, it is preferable that the area ratio of 50% or more of the protruding surface portion 201 is more than 0% and 60% or less, and the area ratio of 70% or more of the protruding surface portion 201 is more than 0% and 20% or less. In this way, even in a state where liquid adheres to the operation reception surface 40a, it is possible to further suppress a decrease in detection accuracy of the operation of the operation reception surface 40a by the operator.

Here, the area ratio of 50% or more and the area ratio of 70% or more of the protruding surface portion 201 described above can be obtained by measuring the area ratio after setting the objective lens to 20 times, the laser brightness to 50%, and the scan pitch to 0.2 µm, and analyzing the threshold setting as a histogram in the area measurement using a 3D measurement laser microscope (Olympus Corporation, 3D MEASURING LASER MICROSCOPE OLS4000).

For comparison, in the case of the above-described satin-like surface, the area ratio of 50% or more of the protruding surface portion as a continuous reference surface is 80% or more. In the case of the above-described satin-like surface, the area ratio of 70% or more of the protruding surface portion as a continuous reference surface is 25% or more. The height 0 to 100% in the case of the satin-like surface is set such that the lowest position among the plurality of recesses in the independent state is 0% and the highest position among the protruding surface portions as the continuous reference surface is 100% per area of 300 µm square. As described above, the area ratio of 50% or more of the protruding surface portion of the satin-like surface is larger than the area ratio of 50% or more of the protruding surface portion 201 of the uneven surface 200 described above. The area ratio of 70% or more of the protruding surface portion of the satin-like surface is also larger than the area ratio of 70% or more of the protruding surface portion 201 of the uneven surface 200 described above. Therefore, in the case of the satin-like surface, the droplets easily coalesce and spread on the protruding surface portion as the continuous reference surface as compared with the above-described uneven surface 200. As a result, the attached liquid tends to form an unintended conductive path. That is, in a case where the operation reception surface is formed of a satin-like surface, if liquid adheres, position information of a detection target is likely to be erroneously detected.

An arithmetic average height Sa (µm) of the uneven surface 200 is preferably 0.82 to 1.44 µm. Here, the arithmetic average height Sa (µm) is a value measured in accordance with ISO 25178 and JIS B0681. Specifically, the measured value can be obtained by using a device such as a 3D measuring laser microscope (Olympus Corporation, 3D MEASURING LASER MICROSCOPE OLS4000) similar to the above, under the measurement conditions as described above, or under the measurement conditions and the measurement method conforming to the standards.

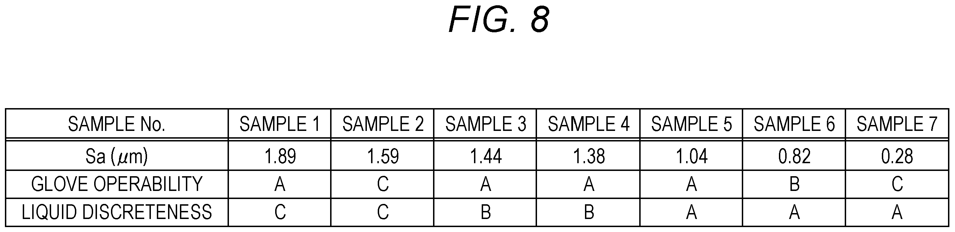

An experiment has been conducted to confirm the influence of the arithmetic average height Sa (µm) on the operability (glove operability) of the operation reception surface 40a in a state where a glove is worn and the liquid discreteness on the operation reception surface 40a. In this experiment, samples 1 to 7 having different arithmetic average heights Sa (µm) have been prepared, and the glove operability and liquid discreteness have been evaluated for these samples 1 to 7. Fig. 8 shows a result of this experiment. The evaluation A illustrated in Fig. 8 means an extremely good result. The evaluation B illustrated in Fig. 8 means a good result but relatively inferior to the evaluation A. The evaluation C illustrated in Fig. 8 means a result that is not good. As illustrated in Fig. 8, by setting the arithmetic average height Sa (µm) to 0.82 to 1.44 µm, good results have been obtained for both the glove operability and the liquid discreteness.

The medical device according to the present disclosure is not limited to the specific configurations illustrated in the above-described embodiments, and various changes and modifications can be made without departing from the scope of the claims. The medical device according to the present disclosure is not limited to the syringe pump shown in the above-described embodiment, and may be, for example, a liquid feeding pump such as an infusion pump, a nutrient pump, or an insulin pump.

### Industrial Applicability

The present disclosure relates to a medical device.

### Reference Signs List

- 1: syringe pump (example of medical device)
- 11: placement portion
- 12: slider
- 13: pusher fixing portion
- 14: clamp
- 20: circuit unit
- 21: communication unit
- 22: clocking unit
- 23: storage unit
- 24: control unit
- 31: input button group
- 32: display unit
- 36: slider drive unit
- 40: operation reception unit
- 40a: operation reception surface
- 41: central portion
- 42: central protruding portion
- 43: peripheral wall
- 44: annular groove
- 46: housing
- 46a: inclined surface portion
- 50: syringe
- 51: cylinder
- 52: hollow portion
- 53: cylinder flange
- 54: outlet hole
- 55: pusher
- 60: position acquisition unit
- 61a to 61h: detection region
- 200: uneven surface
- 201: protruding surface portion
- 202: recessed surface portion
- A: extending direction of cylinder
- B: direction orthogonal to extending direction
- C: circumferential direction around central portion on operation reception surface
- D: first circumferential direction
- E: second circumferential direction
- F: height direction orthogonal to uneven surface
- L: reference surface

## Claims

1. A medical device comprising:
an operation reception unit that includes an operation reception surface;
a position acquisition unit that is capable of acquiring position information based on a position of a detection target on the operation reception surface, the detection target being in contact with or in proximity to the operation reception surface of the operation reception unit; and
a control unit that specifies an operation input by the detection target on a basis of a change in the acquired position information, wherein
the operation reception surface includes an uneven surface having a sea-island structure in which a plurality of protruding surface portions are dispersedly arranged per 1 mm square area.

2. The medical device according to claim 1, wherein the position acquisition unit is a capacitive position detection sensor capable of acquiring the position information of the detection target by detecting a change in capacitance caused by contact or proximity of the detection target to the operation reception surface.

3. The medical device according to claim 1 or 2, wherein
an area ratio of the protruding surface portion at a cross-sectional position of a height of 50% or more of the uneven surface per area of a 300 µm square of the uneven surface is more than 0% and 60% or less, and
the height of 50% or more means a position of 50% or more in a height direction in a case where a lowest position is 0% and a highest position is 100% in the height direction orthogonal to the uneven surface.

4. The medical device according to claim 1 or 2, wherein
an area ratio of the protruding surface portion at a cross-sectional position of a height of 70% or more of the uneven surface per area of a 300 µm square of the uneven surface is more than 0% and 20% or less, and
the height of 70% or more means a position of 70% or more in a height direction in a case where a lowest position is 0% and a highest position is 100% in the height direction orthogonal to the uneven surface.

5. The medical device according to claim 1 or 2, wherein an arithmetic average height of the uneven surface is 0.82 to 1.44 µm.

6. The medical device according to claim 1 or 2, comprising:
a housing that accommodates the position acquisition unit and the control unit, wherein
an outer surface of the housing includes an inclined surface portion extending obliquely with respect to a horizontal direction so as to face an upper side in a vertical direction in a use state, and
the operation reception surface of the operation reception unit is exposed from the inclined surface portion of the housing and extends along the inclined surface portion.

7. The medical device according to claim 1 or 2, wherein
the operation reception surface includes an annular groove having an arcuate cross-sectional shape orthogonal to an extending direction, and
the uneven surface is formed on an inner surface of the annular groove.
